# EUROPEAN PATENT SPECIFICATION

(11) **EP 1 902 666 B1**
(45) Date of publication and mention of the grant of the patent: **18.02.2009**
(21) Application number: 07025045.1
(22) Date of filing: 22.12.2004
(51) Int. Cl.: A61B 1/01, A61B 1/12

(54) **Endoscope apparatus**
Endoskopische Vorrichtung
Appareil d'endoscopie

(30) Priority: 24.12.2003 JP 2003426919
(43) Date of publication of application: 26.03.2008
(62) Divisional of application: 04030522.9
(73) Proprietor: Fujinon Corporation, Saitama-shi, Saitama (JP); SRJ Corporation, Minami-Kawachi-machi Kawachi-gun Tochigi (JP)
(72) Inventor: Takano, Masayuki, Saitama-shi, Saitama (JP)
(74) Representative: Grünecker, Kinkeldey, Stockmair & Schwanhäusser Anwaltssozietät

(56) References cited:
- US-A- 4 040 413
- PATENT ABSTRACTS OF JAPAN vol. 018, no. 301 (C-1210), 9 June 1994 (1994-06-09) -& JP 06 063045 A (OLYMPUS OPTICAL CO LTD), 8 March 1994 (1994-03-08)
- PATENT ABSTRACTS OF JAPAN vol. 2003, no. 02, 5 February 2003 (2003-02-05) & JP 2002 301019 A (YAMAMOTO HIRONORI), 15 October 2002 (2002-10-15)
- PATENT ABSTRACTS OF JAPAN vol. 2000, no. 01, 31 January 2000 (2000-01-31) & JP 11 290263 A (YAMAMOTO HIRONORI), 26 October 1999 (1999-10-26)

## Description

### BACKGROUND OF THE INVENTION

### Field of the Invention

The present invention relates to an endoscope apparatus, and particularly relates to an endoscope apparatus which has a balloon fitted onto an insertion section of an endoscope, and which performs observation by inserting the insertion section into a deep alimentary canal such as a small intestine and a large intestine by alternately inserting an insertion assisting tool covering the insertion section, and the insertion section.

### Description of the Related Art

When the insertion section of an endoscope is inserted into a deep alimentary canal such as a small intestine, by only pushing the insertion section into the deep alimentary canal, the force is difficult to transmit to a tip end of the insertion section due to complicated bending of an intestinal canal, and insertion into a deep part is difficult. For example, if excessive bending and deflection occur to the insertion section, the insertion section cannot be further inserted into a deep part. Thus, there is proposed a method for preventing excessive bending and deflection of the insertion section by inserting the insertion section into a body cavity with an insertion assisting tool covering the insertion section of the endoscope, and guiding the insertion section with the insertion assisting tool.

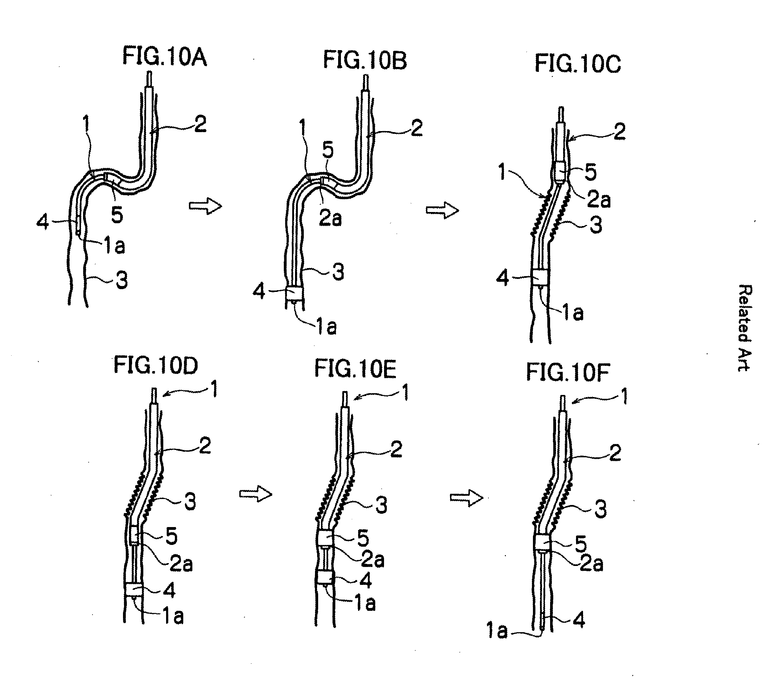

In Japanese Patent Application Laid-open No. 51-11689, an endoscope apparatus provided with a first balloon at a tip end part of an insertion section of the endoscope and provided with a second balloon at a tip end part of the insertion assisting tool (also called an over tube or a sliding tube) is described. According to the endoscope apparatus, an insertion section 1 of an endoscope is first inserted into a intestinal canal 3 with an insertion assisting tool 2 covering the insertion section 1 and a tip end part 1a of the insertion section 1 is inserted into as deep a part as possible, as shown in Fig. 10A. Then, a first balloon 4 fitted onto the tip end part 1a is inflated and fixed to the intestinal canal 3 as shown in Fig. 10B. Next, as shown in Fig. 10C, the insertion section 1 is drawn in hand over hand to remove excessive deflection of the insertion section 1, and makes the insertion section 1 as linear a shape as possible. Next, as shown in Fig. 10D, the insertion assisting tool 2 is pushed through along the insertion section 1, and a tip end part 2a of the insertion assisting tool 2 is disposed near a tip end part 1a of the insertion section 1. Then, as shown in Fig. 10E, a second balloon 5 is inflated, and the tip end part 2a of the insertion assisting tool 2 is fixed to the intestinal canal 3. Next, as shown in Fig. 10F, after the first balloon 4 is deflated, the insertion section 1 is inserted as deep as possible again. On this occasion, the insertion section 1 is guided by the insertion assisting tool 2, and therefore, the insertion section 1 can be smoothly inserted. By repeating the above operation, the tip end part 1a of the insertion section 1 can be inserted into a deep part even in the intestinal canal 3 that is complicatedly bent.

JP 06 063045 A discloses an ultrasound endoscope device comprising an endoscope body with a first balloon, and a short outer tube with a second balloon, being movable over the distal portion of the endoscope body and connected to the latter by two flexible elements, namely a bellows tube and a membrane. The outer tube can be anchored inside a body cavity by means of the second balloon, thereby acting as an insertion assisting tool. The bellows tube prevents contact of the balloon with the outer tube.

### SUMMARY OF THE INVENTION

However, Japanese Patent Application Laid-open No. 51-11689 has the fear that when the insertion section 1 is drawn in, a tip end of the insertion assisting tool 2 contacts the first balloon 4 and damages the first balloon 4.

The present invention is made in view of the above circumstances, and has its object to provide an endoscope apparatus which can prevent a balloon fitted onto an insertion section from contacting an insertion assisting tool and being damaged.

According to the endoscope apparatus of the present invention, the tip end of the insertion assisting tool can be prevented from contacting the balloon by the contact preventing device, and damage to the balloon can be prevented.

### BRIEF DESCRIPTION OF THE DRAWINGS

Fig. 1 is a system schematic diagram showing an embodiment of an endoscope apparatus according to the present invention;
Fig. 2 is a perspective view showing a tip end part of an insertion section in Fig. 1;
Figs. 3A to 3H are explanatory views showing an operation method of the endoscope apparatus in Fig. 1;
Fig. 4 is a schematic view showing a construction of a contact preventing device of an example not forming part of the invention;
Fig. 5 is a schematic view showing a construction of a contact preventing device of an embodiment;
Fig. 6 is a schematic view showing a construction of a different contact preventing device from Fig. 5;
Fig. 7 is a schematic view showing a construction of a contact preventing device of an example not forming part of the invention;
Fig. 8 is a schematic view showing a construction of a contact preventing device with a different detection position from Fig. 7;
Fig. 9 is a schematic view showing a construction of a contact preventing device of an example not forming part of the invention; and
Figs. 10A to 10F are explanatory views showing an operation method of a conventional endoscope apparatus.

### DETAILED DESCRIPTION OF THE PREFERRED EMBODIMENTS

Preferred embodiments of an endoscope apparatus according to the present invention will be explained in accordance with the attached drawings hereinafter.

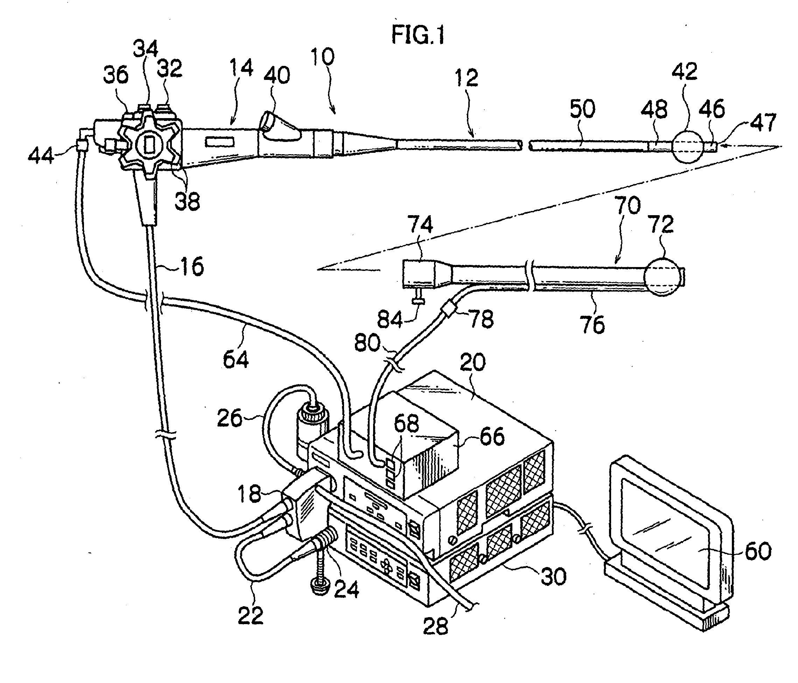

Fig. 1 shows a system schematic diagram showing an embodiment of an endoscope apparatus according to the present invention. The endoscope apparatus shown in Fig. 1 is mainly constructed by an endoscope 10, a light source device 20, a processor 30, a balloon control device 66 and an insertion assisting tool 70.

The endoscope 10 comprises an insertion section 12 which is inserted into a body cavity and a hand operation section 14 connected to the insertion section 12, and a universal cable 16 is connected to the hand operation section 14. An LG connector 18 is provided at a tip end of the universal cable 16, and the LG connector 18 is connected to the light source device 20. An electric connector 24 is connected to the LG connector 18 via a cable 22, and the electric connector 24 is connected to the processor 30. An air/water supply tube 26 for supplying air and water, and a suction tube 28 for sucking air are connected to the LG connector 18.

On the hand operation section 14, an air/water passing button 32, a suction button 34 and a shutter button 36 are provided in parallel, and a pair of angle knobs 38 and 38, and a forceps insertion part 40 are provided. Further, a supply/suction port 44 for supplying fluid to a first balloon 42 which will be described later and sucking fluid from the first balloon 42 is provided at a base end part of the hand operation section 14. The explanation will be made hereinafter with the example using air as the fluid, but the other fluid, for example, inert gas and water may be used.

The insertion section 12 is constructed by a tip end part 46, a curving part 48 and a flexible part 50, the curving part 48 is remotely operated to curve by rotating a pair of angle knobs 38 and 38 provided on the hand operation section 14. Thereby, a tip end surface 47 of the tip end part 46 can be directed to a desired direction.

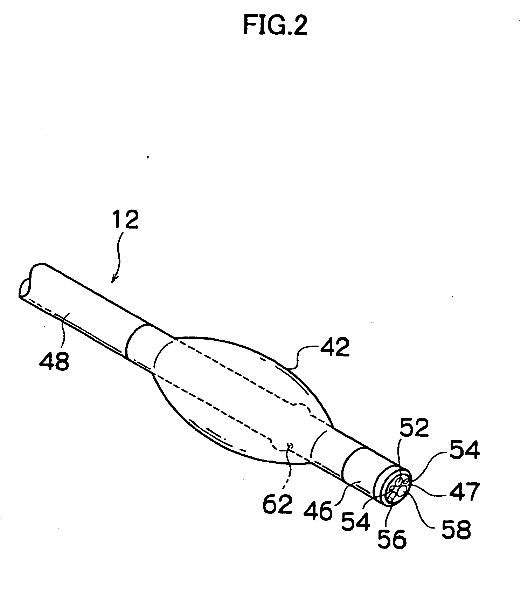

As shown in Fig. 2, the tip end surface 47 of the tip end part 46 is provided with an observation optical system 52, an illumination optical system 54, an air/water passing nozzle 56, a forceps port 58 and the like. CCD (not shown) is placed behind the observation optical system 52, and a signal cable is connected to the base board supporting the CCD. The signal cable is provided to extend to the electric connector 24 by being inserted through the insertion section 12, the hand operation section 14 and the universal cable 16 in Fig. 1, and is connected to the processor 30. Accordingly, an observed image taken by the observation optical system 52 is formed on a light-receiving surface of the CCD, and converted into an electrical signal, and the electrical signal is outputted to the processor 30 via the signal cable, and converted into a picture signal. Thereby, the observed image is displayed on a monitor 60 connected to the processor 30.

An emission end of a light guide (not shown) is placed behind the illumination optical systems 54 in Fig. 2. The light guide is inserted through the insertion section 12, the hand operation section 14 and the universal cable 16 in Fig. 1, and an incident end is placed at the LG connector 18. Thereby, the illumination light which is irradiated from the light source device 20 is transmitted to the illumination optical systems 54 via the light guide, and is irradiated from the illumination optical systems 54.

The air/water passing nozzle 56 (see Fig. 2) is inserted through a valve (not shown) which is operated by the air/water passing button 32, and is further inserted through the air/water supplying tube 26. Accordingly, by operating the air/water passing button 32, air or water is injected toward the observation optical system 52 from the air/water passing nozzle 56.

The forceps port 58 (see Fig. 2) is inserted through the forceps insertion part 40, and is inserted through a valve (not shown) which is operated by the suction button 34, and further inserted through the suction tube 28. Accordingly, by operating the suction button 34, a diseased portion and the like are sucked from the forceps port 58, and by inserting an operative instrument from the forceps insertion part 40, the operative instrument is led out of the forceps port 58.

As shown in Fig. 2, the first balloon 42 made of an elastic body such as rubber is fitted onto an outer peripheral surface of the insertion section 12. The first balloon 42 is formed in a substantially cylindrical shape with both end portions narrowed down, and is fitted by fixing both the end portions of the first balloon 42 to the insertion section 12 after the insertion section 12 is inserted through the first balloon 42. As for the fixing method of both the end portions of the first balloon 42, for example, a thread is wound around both the end portions of the first balloon 42, for example, and the first balloon 42 is closely fitted onto the entire periphery of the outer peripheral surface of the insertion section 12. Instead of winding the thread, fixing rings may be fitted onto both the end portions of the first balloon 42.

An air port 62 is formed at the attaching position of the first balloon 42 to the insertion section 12. The air port 62 communicates with the supply/suction port 44 of the hand operation section 14 in Fig. 1. A tube 64 is connected to the supply/suction port 44 and the tube 64 is connected to the balloon control device 66. The balloon control device 66 is a device which supplies air to the first balloon 42 and sucks air via the tube 64, and controls the air pressure on the supply and suction of air, and is operated by an operation button 68 provided at a front surface. The first balloon 42 is inflated in a substantially spherical shape by supplying air, and attached onto the outer surface of the insertion section 12 by sucking air.

Meanwhile, the insertion assisting tool 70 is formed in a cylindrical shape, and has a slightly larger inner diameter than the outer diameter of the insertion section 12, and includes sufficient flexibility. A rigid gripping portion 74 is provided at a base end of the insertion assisting tool 70, and the insertion section 12 is inserted from the gripping part 74.

A balloon air port 78 is provided at a base end side of the insertion assisting tool 70. An air supply tube 76 with an inner diameter of about 1 mm is connected to the balloon air port 78, and the air supply tube 76 is bonded to an outer peripheral surface of the insertion assisting tool 70, and provided to extend to the tip end part of the insertion assisting tool 70.

A second balloon 72 made of latex is fitted to the vicinity of the tip end of the insertion assisting tool 70. The second balloon 72 is formed into a substantially cylindrical shape with both ends narrowed, and is fitted onto the insertion assisting tool 70 with the insertion assisting tool 70 penetrated through the second balloon 72. The aforementioned tube 76 is opened inside the second balloon 72, and the second balloon 72 communicates with the balloon air port 78 via the tube 76. A tip end of a tube 80 is connected to the balloon air port 78, and a base end of the tube 80 is connected to the aforementioned balloon control device 66. Accordingly, when air is supplied to the balloon air port 78 by the balloon control device 66, air is blown into the second balloon 72, whereby the second balloon 72 is inflated. When air is sucked from the balloon air port 78, air is sucked from the second balloon 72, whereby the second balloon 72 is deflated. Reference numeral 84 in Fig. 1 designates an inlet port for filling a lubricant such as water into the insertion assisting tool 70.

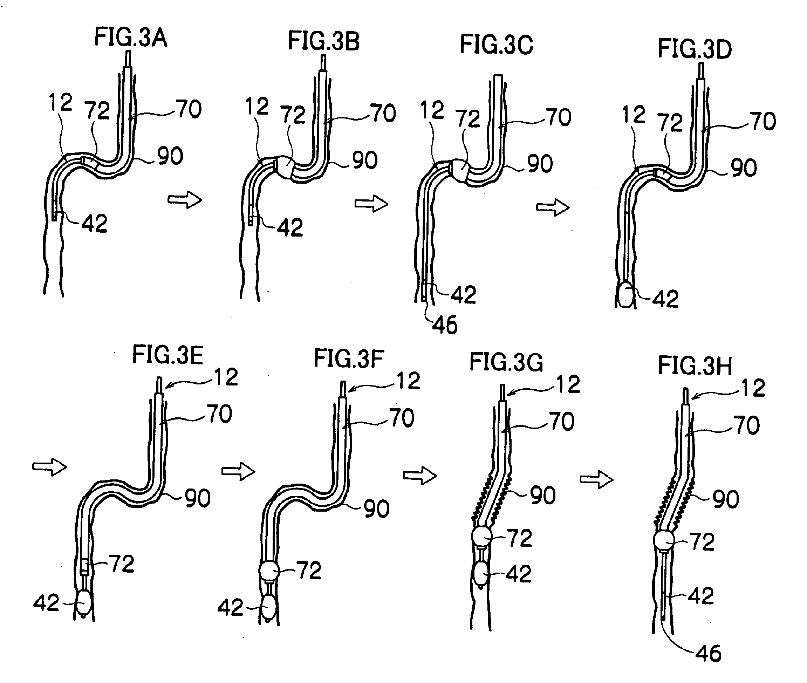

Next, an operation method of the endoscope apparatus constructed as described above will be explained in accordance with Figs. 3A to 3H.

First, as shown in Fig. 3A, the insertion section 12 is inserted into an intestinal canal (for example, descending limb of duodenum) in the state in which the insertion assisting tool 70 covers the insertion section 12. At this time, the first balloon 42 and the second balloon 72 are deflated.

Next, as shown in Fig. 3B, in the state in which the tip end of the insertion assisting tool 70 is inserted into a bent portion of the intestinal canal 90, air is supplied to the second balloon 72 to inflate the second balloon 72. As a result, the second balloon 72 is caught by the intestinal canal 90, and the tip end of the insertion assisting tool 70 is fixed to the intestinal canal 90.

Next, as shown in Fig. 3C, only the insertion section 12 of the endoscope 10 is inserted to a deep part of the intestinal canal 90 (insertion operation). Then, as shown in Fig. 3D, air is supplied to the first balloon 42 to inflate the first balloon 42. As a result, the first balloon 42 is fixed to the intestinal canal 90 (fixing operation).

Next, after air is sucked from the second balloon 72 to deflate the second balloon 72, the insertion assisting tool 70 is pushed in, and inserted along the insertion section 12, as shown in Fig. 3E (pushing operation). Then, after the tip end of the insertion assisting tool 70 is pushed into the vicinity of the first balloon 42, air is supplied to the second balloon 72 to inflate the second balloon 72 as shown in Fig. 3F. As a result, the second balloon 72 is fixed to the intestinal canal 90. Namely, the intestinal canal 90 is gripped by the second balloon 72 (gripping operation).

Next, as shown in Fig. 3G, the insertion assisting tool 70 is drawn in (drawing operation). Thereby, the intestinal canal 90 is in the contracted state, and excessive deflection and bending of the insertion assisting tool 70 are eliminated.

Next, as shown in Fig. 3H, air is sucked from the first balloon 42 to deflate the first balloon 42. Then, the tip end part 46 of the insertion section 12 is inserted into as deep a part of the intestinal canal 90 as possible. Namely, the inserting operation as shown in Fig. 3C is performed again. Thereby, the tip end part 46 of the insertion section 12 can be inserted into a deep part of the intestinal canal 90. When the insertion section 12 is further inserted into a deep part, the pushing operation as shown in Fig. 3E is performed after the fixing operation as shown in Fig. 3D is performed, the gripping operation as shown in Fig. 3F and the drawing operation as shown in Fig. 3G, and the inserting operation as shown in Fig. 3H are repeatedly performed in sequence. Thus, the insertion section 12 can be further inserted into a deep part of the intestinal canal 90.

Incidentally, the endoscope apparatus according to the present invention is provided with a contact preventing device which prevents the insertion assisting tool 70 from contacting the first balloon 42 when the pushing operation shown in Fig. 3E is performed.

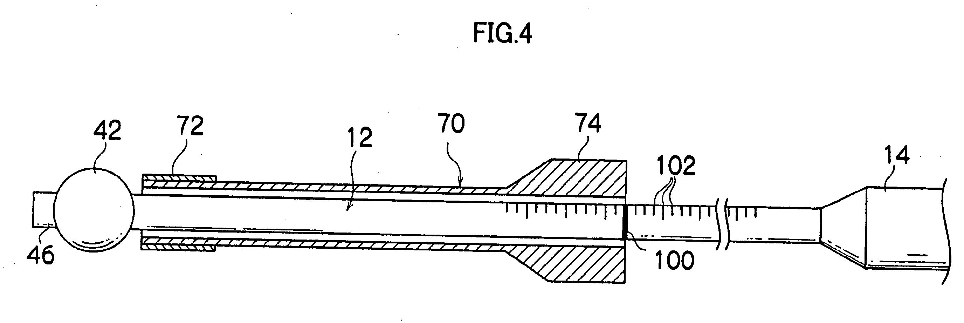

Fig. 4 is a schematic view showing a construction of an example of the contact preventing device, not forming part of the invention.

A contact prevention indicator 100 for contact prevention is formed on the outer peripheral surface of the insertion 12 shown in Fig. 4. The contact prevention indicator 100 is formed at the position where it appears from the base end side of the insertion assisting tool 70 when the tip end of the insertion assisting tool 70 is in the state in which the tip end of the insertion assisting tool 70 approaches the first balloon 42, but does not contact the first balloon 42 (hereinafter, called a limit state) as shown in Fig. 4. When scales 102 and the like are formed on the insertion section 12, it is preferable to form the contact prevention indicator 100 to be different in thickness, color, shape and the like from the scale 102 to be recognized as the contact prevention indicator 100 at a glance. For example, it is preferable to form the contact prevention indicator 100 to be thicker than the scale 102 or form it in a different color from the scale 102.

In the first embodiment constructed as described above, when the insertion assisting tool 70 is pushed to the tip end side of the insertion section 12 and is brought into the limit state, the indicator 100 appears from the base end side of the insertion assisting tool 70. Accordingly, the operator recognizes the indicator 100, and thereby, the operator can prevent the tip end of the insertion assisting tool 70 from contacting the first balloon 42. Thereby, the first balloon 42 can be prevented from being damaged.

The aforementioned example is provided with the indicator 100 indicating the limit state, but other than the indicator, an indicator indicating that the tip end of the insertion assisting tool 70 approaches the limit state may be provided. This makes it possible to recognize that the tip end of the insertion assisting tool 70 approaches the first balloon 42 stepwise.

In the aforementioned example, the contact prevention indicator 100 is formed separately from the scales 102, but the scale 102 corresponding to the position of the indicator 100 may be changed from the other scales 102 in thickness, color, shape and the like. For example, the other scales 102 may be formed in white, and the scale 102 at the position corresponding to the contact prevention indicator 100 may be formed in red.

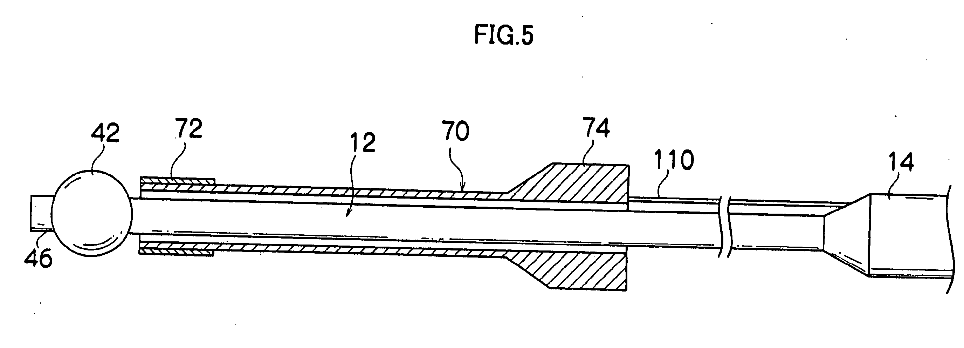

Fig. 5 is a schematic view showing a construction of an embodiment of the contact preventing device.

A string-shaped member 110 shown in Fig. 5 has its both ends respectively connected to the insertion assisting tool 70 and the hand operation section 14. As the string-shaped member 110, a chain, a string or the like, which is not extended in the axial direction is used, and the maximum length of the string-shaped member 110 is set at the distance between the insertion assisting tool 70 and the hand operation section 14 in the limit state. It is suitable to connect the both ends of the string-shaped member 110 to the insertion assisting tool 70 and the hand operation section 14 after covering the insertion section with the insertion assisting tool 70.

According to the embodiment constructed as described above, when the insertion assisting tool 70 is pushed to the tip end side of the insertion section 12, the string-shaped member 110 strains when the insertion assisting tool 70 is in the limit state, and the insertion assisting tool 70 cannot be pushed any more. Accordingly, the tip end of the insertion assisting tool 70 can be prevented from contacting the first balloon 42.

In the aforementioned embodiment, the base end side of the string-shaped member 110 is connected to the hand operation section 14, but the base end side of the string-shaped member 110 may be connected to the base end part of the insertion section 12.

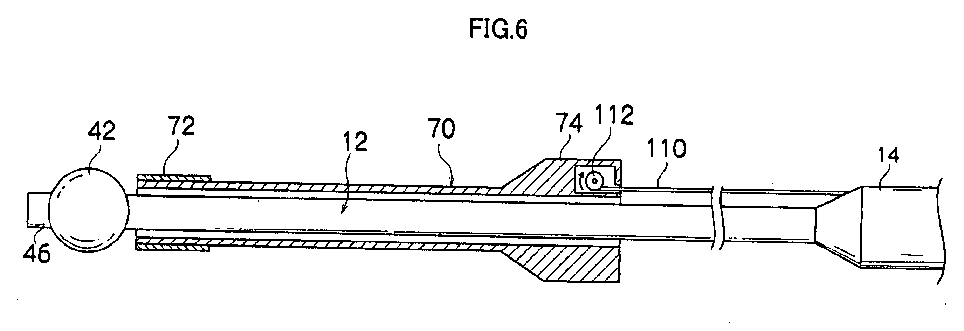

As shown in Fig. 6, an end portion of the string-shaped member 110 is wound around a take-up roller 112, and thereby, excessive deflection of the string-shaped member 110 may be removed. The taken-up roller 112 shown in Fig. 6 is provided inside the gripping part 74 of the insertion assisting tool 70, and is biased in the arrow direction by a biasing device not shown. Accordingly, the string-shaped member 110 which is loosened between the insertion assisting tool 70 and the insertion section 14 is automatically wound by the taken-up roller 112. Therefore, it can be prevented that the loosened string-shaped member 110 interferes with the operation. The taken-up roller 112 may be provided at the hand operation section 14 side. In the apparatus shown in Fig. 6, the rotational frequency of the taken-up roller 112 is detected, and when the rotational frequency becomes a set value, it is determined as the limit state, and the rotation of the taken-up roller 112 may be stopped. Alternatively, when the rotational frequency becomes the set value, the warning may be sent out.

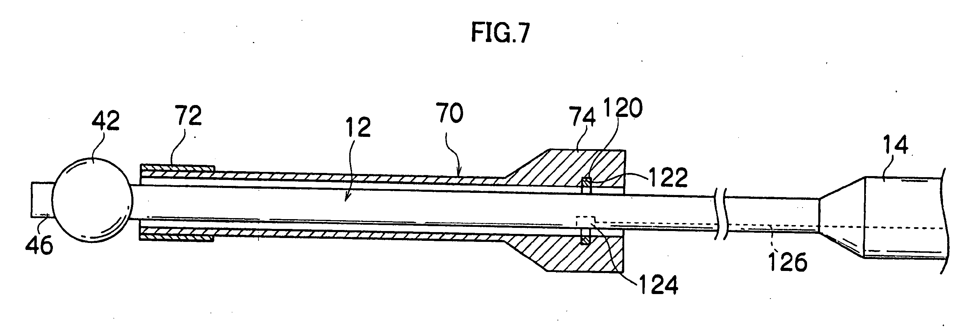

Fig. 7 is a schematic view showing a construction of another example of the contact preventing device, not forming part of the invention.

A recessed groove 120 is formed in an inner peripheral surface of the insertion assisting tool 70 shown in Fig. 7, and a ring-shaped reflector plate 122 is embedded in and fixed to the recessed groove 120. The reflector plate 122 is disposed to be detected by an optical sensor 124 when the insertion assisting tool 70 is in the limit state.

The optical sensor 124 is fixed to the insertion section 12. The optical sensor 124 irradiates light from the outer peripheral surface of the insertion section 12 and receives the reflected light, and thereby, detects the change in the reflected light amount. Accordingly, if the reflector plate 122 is disposed at the outer side of the optical sensor 124, the reflected light amount changes, and therefore, the optical sensor 124 can detect the reflector plate 122.

A cable 126 is connected to the optical sensor 124, and the cable 126 is connected to the processor 30 in Fig. 1. The processor 30 performs warning display on the monitor 60, or issues warning sound from a speaker built in the monitor 60 when the optical sensor 124 detects the reflector plate 122.

According to the example constructed as described above, when the insertion assisting tool 70 is pushed to the tip end side of the insertion section 12, the optical sensor 124 detects the reflector plate 122 when the insertion assisting tool 70 comes to the limit state, and warning sound is issued. Accordingly, the tip end of the insertion assisting tool 70 can be prevented- from contacting the first balloon 42.

In the aforementioned example, the reflector plate 122 is provided as the detected member to be detected by the optical sensor 124, but the member to be detected is not limited to this, and may be an indicator for optical detection or the like.

In the aforementioned example, the reflector plate 122 is provided at the insertion assisting tool 70, and the optical sensor 124 is provided at the insertion section 12, but in reverse, the reflector plate 122 may be provided at the outer peripheral surface of the insertion section 12, and the optical sensor 124 may be provided at an inner peripheral surface of the insertion assisting tool 70. In this case, it is suitable to provide the display part on the outer peripheral surface of the gripping part 74 of the insertion assisting tool 70 and display a warning message.

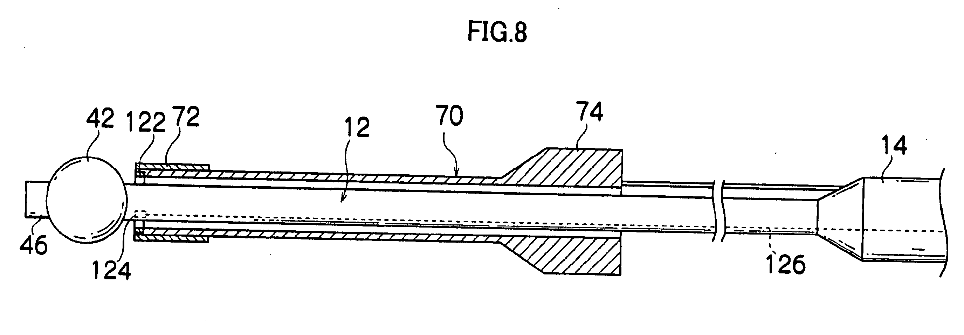

In the aforementioned example, the reflector plate 122 is provided at the gripping part 74 at the base end of the insertion assisting tool 70, but the attaching position of the member to be detected such as the reflector plate 122 is not limited to this. For example, as shown in Fig. 8, the reflector plate 122 may be provided at the tip end of the insertion assisting tool 70, and the optical sensor 124 may be placed to detect the reflector plate 122 in the limit state. In this case, the tip end position of the insertion assisting tool 70 is detected, and therefore, the tip end of the insertion assisting tool 70 can be more reliably prevented from contacting the first balloon 42.

Further, in the aforementioned example, warning is issued in the limit state, but it can be detected that the tip end of the insertion assisting tool 70 approaches the limit state by providing a plurality of reflector plates 122, and warning can be issued. Thereby, stepwise warning can be performed.

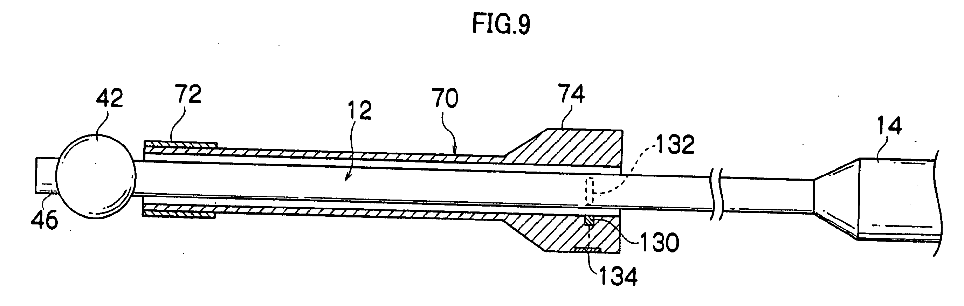

Fig. 9 is a schematic view showing a construction of another example of the contact preventing device, not forming part of the invention.

A magnetic body 132 is provided inside the insertion section 12 shown in Fig. 9. The magnetic body 132 is disposed at the position which is detected by a magnetic sensor 130 which will be described later when the insertion assisting tool 70 is in the limit state. The magnetic body 132 is formed into a ring shape, and is disposed in the state in which the content such as the aforementioned light guide and tube is inserted through the magnetic body 132. The shape, construction and the like of the magnetic body 132 are not especially limited, and for example, a magnetic tape or the like may be used.

Meanwhile, the magnetic sensor 130 is provided at the gripping part 74 of the insertion assisting tool 70. When the magnetic sensor 130 detects the magnetic body 132, a message of warning is displayed on the display part 134 provided on the outer surface of the gripping part 74.

According to the example constructed as described above, when the insertion assisting tool 70 is pushed to the tip end side of the insertion section 12, the magnetic sensor 130 detects the magnetic body 132 and the message of warning is displayed on the display part 134, when the insertion assisting tool 70 is in the limit state. Accordingly, the tip end of the insertion assisting tool 70 can be prevented from contacting the first balloon 42.

In the aforementioned example, the magnetic body 132 is provided at the insertion section 12, and the magnetic sensor 130 is provided at the insertion assisting tool 70, but in reverse, the magnetic body 132 may be provided at the insertion assisting tool 70, while the magnetic sensor 130 may be provided at the insertion section 12. The attaching positions of the magnetic body 132 and the magnetic sensor 130 are not limited to the base end of the insertion assisting tool 70, but may the tip end of the insertion assisting tool 70, for example. Thereby, the tip end of the insertion assisting tool 70 can be prevented from contacting the first balloon 42 more reliably.

The aforementioned example is constructed to issue warning in the limit state, but by providing a plurality of magnetic bodies 132, it may be detected that the insertion assisting tool 70 approaches the limit state and warning may be issued. Thereby, stepwise warning can be performed.

The aforementioned embodiment cites the example of a double balloon type endoscope apparatus with the first balloon 42 fitted to the insertion section 12 and the second balloon 72 fitted to the insertion assisting tool 70, but it is suitable if only the first balloon 42 is fitted to the insertion section 12, and the present invention can be also applied to the endoscope apparatus in which the second balloon 72 is not fitted to the insertion assisting tool 70.

The operation methods of the endoscope apparatuses in the aforementioned embodiment are not limited to the procedure shown in Fig. 3, and the effect of the present invention can be also obtained by the operation method shown in Fig. 10. Namely, by providing the aforementioned contact preventing device, the insertion assisting tool 2 can be prevented from contacting the first balloon 4 and the first balloon 4 can be prevented from being damaged, when the insertion assisting tool 2 is pushed in along the insertion section 1 as shown in Fig. 10C.

In the aforementioned examples, warning is performed when the limit state is detected, but the insertion assisting tool 70 may be prevented from contacting the first balloon 42 by fixing the insertion part 12 and the insertion assisting tool 70. For example, the elastic member made of rubber or the like is provided to be able to protrude and retreat with respect to the inner peripheral surface of the insertion assisting tool 70, and the elastic member is controlled to protrude when the limit sated is detected. Thereby, the elastic member protrudes and abuts to the outer peripheral surface of the insertion section 12 when the insertion assisting tool 70 is in the limit state, and the insertion section 12 and the insertion assisting tool 70 are fixed. Thereby, the insertion assisting tool 70 can be reliably prevented from contacting the first balloon 42.

## Claims

1. An endoscope apparatus comprising:
an endoscope (10) with an insertion section (12) being connected to a hand operation section (14) and a balloon (42) fitted to the insertion section,
an insertion assisting tool (70) which covers the insertion section and guides insertion of the insertion section into a body cavity; and
a contact preventing device which prevents a tip end of the insertion assisting tool (70) from contacting the balloon (42),
**characterized in that**
the contact preventing device is a string-shaped member (110) which connects the insertion assisting tool (70) and the hand operation section (14).

## Patentansprüche

1. Endoskopie-Vorrichtung umfassend:
ein Endoskop (10) mit einem Einführabschnitt (12), der mit einem Handbetätigungsabschnitt (14) und einem an den Einführabschnitt angepassten Ballon (42) verbunden ist;
ein Einführhilfswerkzeug (70), das den Einführabschnitt bedeckt und die Einführung des Einführabschnitts in eine Körperhöhle führt; und
eine Kontaktverhinderungsvorrichtung, die verhindert, dass ein spitzenseitiges Ende des Einführhilfswerkzeugs (70) den Ballon (42) berührt,
**dadurch gekennzeichnet, dass**
die Kontaktverhinderungsvorrichtung ein schnurförmiges Element (110) ist, das das Einführhilfswerkzeug (70) und den Handbetätigungsabschnitt (14) verbindet.

## Revendications

1. Appareil d'endoscopie, comprenant :
un endoscope (10) avec une section d'insertion (12) qui est raccordée à une section de commande manuelle (14) et un ballonnet (42) monté sur la section d'insertion,
un outil d'aide à l'insertion (70) qui recouvre la section d'insertion et guide l'insertion de la section d'insertion dans une cavité corporelle ; et
un dispositif anti-contact qui empêche une extrémité de pointe de l'outil d'aide à l'insertion (70) d'entrer en contact avec le ballonnet (42),
**caractérisé en ce que :**
le dispositif anti-contact est un élément en forme de ficelle (110) qui raccorde l'outil d'aide à l'insertion (70) et la section de commande manuelle (14).
